# EUROPEAN PATENT SPECIFICATION

(11) **EP 3 806 726 B1**
(45) Date of publication and mention of the grant of the patent: **08.11.2023**
(21) Application number: 19733374.3
(22) Date of filing: 10.06.2019
(51) Int. Cl.: A61B 5/022, A61B 5/021

(54) **FINGER CUFF BLOOD PRESSURE MEASUREMENT SYSTEM INCLUDING A HEART REFERENCE SENSOR**
FINGERMANSCHETTEN-BLUTDRUCKMESSSYSTEM MIT EINEM HERZREFERENZSENSOR
SYSTÈME À MANCHON DE DOIGT DE MESURE DE TENSION ARTÉRIELLE COMPRENANT UN CAPTEUR DE RÉFÉRENCE CARDIAQUE

(30) Priority: 12.06.2018 US 201862683798 P; 24.05.2019 US 201916422696
(43) Date of publication of application: 21.04.2021
(73) Proprietor: Edwards Lifesciences Corporation, Irvine, CA 92614 (US)
(72) Inventor: LEE, Jeong, Soo, Irvine, CA 92614 (US); GUELEN, Ilja, Irvine, CA 92614 (US)
(74) Representative: Eisenführ Speiser
(86) International application number: PCT/US2019/036268
(87) International publication number: WO 2019/241102

(56) References cited:
- EP-A1- 2 502 555
- EP-A1- 3 213 782
- WO-A2-2012/021731
- US-A- 4 779 626
- US-A1- 2010 044 260

## Description

### BACKGROUND

### Field

Embodiments of the invention relate to non-invasive blood pressure measurement using a finger cuff and a volume clamp method with a heart reference sensor. In particular, embodiments of the invention relate to a heart reference sensor that utilizes non-plasticized tubing or chemically inert tubing.

### Relevant Background

There are presently many different types of pressure sensor configurations for measuring blood pressure and blood pressure waveforms. As one example, a finger cuff blood pressure measurement system utilizing a finger cuff with a pressure sensor may be used to measure blood pressure utilizing the volume clamp method.

Volume clamping is a technique for non-invasively measuring blood pressure in which pressure is applied to a subject's finger in such a manner that arterial pressure may be balanced by a time varying pressure to maintain a constant arterial volume. In a properly fitted and calibrated system, the applied time varying pressure is equal to the arterial blood pressure in the finger. The applied time varying pressure may be measured to provide a reading of the patient's arterial blood pressure. This may be accomplished by a finger cuff that is arranged around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter and indicative of the pressure in the artery. In the finger cuff implementation, by inflating the bladder in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder such that the diameter of the finger artery is kept constant, the blood pressure may be monitored in very precise detail as the pressure in the inflatable bladder is directly linked to the blood pressure.

In a typical present-day finger cuff implementation, a volume clamp system is used with the finger cuff. The volume clamp system typically includes a pressure generating system and a regulating system that includes: a pump, a valve, and a pressure sensor in a closed loop feedback system that is used in conjunction with the measurement of the arterial volume. To accurately measure blood pressure, the feedback loop provides sufficient pressure generating and releasing capabilities to match the pressure oscillations of the subject's blood pressure.

Further, a second pressure sensor, a heart reference sensor (HRS), may be utilized with the finger cuff system to compensate for pressures generated by height differences between the patient's finger and heart. The HRS connects a liquid filled bladder located at the patient's heart level to a pressure sensor located at the patient's finger or wrist unit through a liquid filled tube. The gravity generated pressures between the patient's heart level and finger level are measured by the HRS and subtracted from the finger cuff pressure sensor in the system's data processing software/algorithms.

In present HRS systems, flexible polyvinyl chloride (PVC) tubing is often filled with bioblend, biodegradable oil formulated from renewable base stocks. This biodegradable oil is used to provide pressure readings at different elevations to enable zeroing at the patient's heart level for non-invasive pressure monitoring systems, such as, for finger cuff blood pressure measurement systems.

Unfortunately, flexible PVC tubing contains a significant amount of plasticizer. Bioblend oil and plasticizer are miscible and over time, plasticizer migrates into oil, causing excess oil-plasticizer mixture inside the tubing. Chemical analysis has shown plasticizer can migrate into oil resulting in fluid mixture having 80% oil and 20% plasticizer. Therefore, within the closed HRS fluid line, up to a 25% fluid volume increase can occur. Excess oil-plasticizer mixture inside the tubing causes bulging of the bladder of the HRS over time resulting in drift and difficulty in the zeroing functions of the HRS. As bulging of the bladder becomes more severe, the bladder reaches stretchability (elastic) limit and in effect becomes a taut rigid member. Such "rigid" member imparts step function like back pressure to the sensor with any slight perturbation such as lowering the height of the bladder relative to the sensor height thus causing out of range issues.
EP 2 502 555 A1 is concerned with a method of determining the oxygen delivery and comprises the steps of non-invasive measurement of the arterial blood pressure waveform, determination of cardiac output from a pulse wave analysis of said blood pressure waveform, non-invasive measurement of oxygen saturation and hemoglobin concentration and real time calculation of oxygen delivery based on the measured quantities. US4779626 discusses blood pressure monitoring from a finger, taking hydrostatic pressure into consideration.

### BRIEF DESCRIPTION OF THE DRAWINGS

FIG. 1 is a diagram of an optional example of a blood pressure measurement system.
FIG. 2 is a block diagram illustrating a finger cuff and a pressure generating and regulating system, according to an optional example.
FIG. 3 is a diagram illustrating a finger cuff system with a heart reference sensor (HRS) and tubing, according to an optional example.
FIG. 4 is a diagram illustrating an HRS and tubing, according to an optional example.
FIG. 5 is a diagram illustrating an HRS and tubing, according to an optional example.

### DETAILED DESCRIPTION

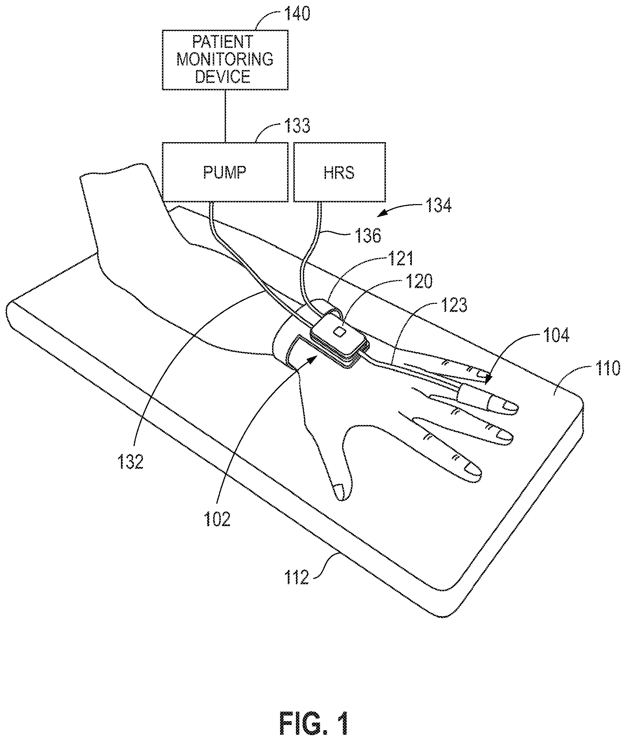

With reference to Figure 1, which illustrates an optional example of a blood pressure measurement system, a blood pressure measurement system 102 that includes a finger cuff 104 that may be attached to a patient's finger and a blood pressure measurement controller 120, which may be attached to the patient's body (e.g., a patient's wrist or hand) is shown.

The blood pressure measurement system 102 may further be connected to a patient monitoring device 140, and, in some optional examples, a separate pump 133. Further, in some optional examples, finger cuff 104 may include a bladder (not shown) and an LED-PD pair (not shown), which are conventional for finger cuffs, as will be described in more detail later.

In one optional example, the blood pressure measurement system 102 may include a pressure measurement controller 120 that includes: a small internal pump, a small internal valve, a pressure sensor, and control circuitry. In this optional example, the control circuitry may be configured to: control the pneumatic pressure applied by the internal pump to the bladder of the finger cuff 104 to replicate the patient's blood pressure based upon measuring the pleth signal received from the LED-PD pair of the finger cuff 104. Further, the control circuitry may be configured to: control the opening of the internal valve to release pneumatic pressure from the bladder; or the internal valve may simply be an orifice that is not controlled. Additionally, the control circuitry may be configured to: measure the patient's blood pressure by monitoring the pressure of the bladder based upon the input from a pressure sensor, which should be related to or the same as patient's blood pressure, and may display the patient's blood pressure on the patient monitoring device 140.

In another optional example, a conventional pressure generating and regulating system may be utilized, in which, a pump 133 is located remotely from the body of the patient. In this optional example, the blood pressure measurement controller 120 receives pneumatic pressure from remote pump 133 through tube 132 and passes on the pneumatic pressure through tube 123 to the bladder of finger cuff 104. Blood pressure measurement device controller 120 may also control the pneumatic pressure (e.g., utilizing a controllable valve) applied to the finger cuff 104 as well as other functions. In this optional example, the pneumatic pressure applied by the pump 133 to the bladder of finger cuff 104 to replicate the patient's blood pressure based upon measuring the pleth signal received from the LED-PD pair of the finger cuff 104 (e.g., to keep the pleth signal constant) and measuring the patient's blood pressure by monitoring the pressure of the bladder may be controlled by the blood pressure measurement controller 120 and/or a remote computing device and/or the pump 133 and/or the patient monitoring device 140 to implement the volume clamping method. In some optional examples, a blood pressure measurement controller 120 is not used at all and there is simply a connection from tube 132 from a remote pump 133 including a remote pressure regulatory system to finger cuff 104, and all processing for the pressure generating and regulatory system, data processing, and display is performed by a remote computing device.

Also, in one optional example, a heart reference sensor (HRS) 134 may be utilized with the finger cuff system 102 to compensate for pressures generated by height differences between the patient's finger and heart. The HRS 134 may connect a liquid filled bladder located at the patient's heart level to a pressure sensor located at the patient's finger or at the blood pressure measurement controller 120 at the wrist, or at other locations near the patient's finger, through a liquid filled tube 136. The gravity generated pressures between the patient's heart level and finger level are measured by the HRS 134 and subtracted from the finger cuff pressure sensor in the system's data processing software/algorithms. Continuing with this optional example, the blood pressure measurement controller 120 utilizing the volume clamp method: controls the pneumatic pressure applied to the bladder of finger cuff 104 to replicate the patient's blood pressure based upon measuring the pleth signal received from the LED-PD pair of the finger cuff 104 (e.g., to keep the pleth signal constant); measures the patient's blood pressure by monitoring the pressure of the bladder; and subtracts out the gravity generated pressure differences between the patient's heart level and finger level as measured by the HRS 134 in calculating the patient's blood pressure. Aspects of this optional example will be described in more detail hereafter.

Continuing with this optional example, as shown in Figure 1, a patient's hand may be placed on the face 110 of an arm rest 112 for measuring a patient's blood pressure with the blood pressure measurement system 102. The blood pressure measurement controller 120 of the blood pressure measurement system 102 may be coupled to a bladder of the finger cuff 104 in order to provide pneumatic pressure to the bladder for use in blood pressure measurement. Further, blood pressure measurement controller 120 may be coupled to the patient monitoring device 140. Also, in an optional example, as previously described, blood pressure measurement controller 120 may be coupled to a remote pump 133 through tube 132 to receive pneumatic pressure for the bladder of the finger cuff 104. The patient monitoring device 140 may be any type of medical electronic device that may read, collect, process, display, etc., physiological readings/data of a patient including blood pressure, as well as any other suitable physiological patient readings. Data may be transmitted to and from the blood pressure measurement controller 120 to the patient monitoring device 140.

As an optional example, as can be seen in Figure 1, the finger cuff 104 may be attached to a patient's finger and the blood pressure measurement controller 120 may be attached on the patient's hand or wrist with an attachment bracelet 121 that wraps around the patient's wrist or hand. The attachment bracelet 121 may be metal, plastic, Velcro, etc. It should be appreciated that this is just one optional example of attaching a blood pressure measurement controller 120 and that any suitable way of attaching a blood pressure measurement controller to a patient's body or in close proximity to a patient's body may be utilized and that, in some optional examples, a blood pressure measurement controller 120 may not be used at all. It should further be appreciated that the finger cuff 104 may be connected to a blood pressure measurement controller described herein, or a pressure generating and regulating system of any other kind, such as a pressure generating and regulating system that is located remotely from the body of the patient. Any kind of pressure generating and regulating system can be used, including but not limited to the blood pressure measurement controller, and may be described simply as a pressure generating and regulating system that may be used with a finger cuff 104 including an LED-PD pair and a bladder to implement the volume clamping method.

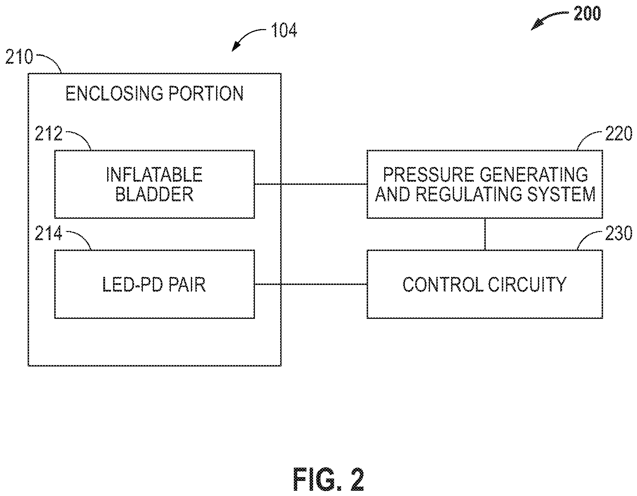

With additional reference to Figure 2, Figure 2 is a block diagram illustrating a finger cuff and a pressure generating and regulating system, according to an optional example. As an optional example, as shown in Figure 2, finger cuff 104 may include an enclosing portion 210, an inflatable bladder 212 and an LED-PD pair 214. The enclosing portion 210 may encircle or enclose a patient's finger and include the inflatable bladder 212, and the LED-PD pair 214. The inflatable bladder 212 may be pneumatically connected to a pressure generating and regulating system 220. The LED may be used to illuminate the finger skin and light absorption or reflection may be detected with the PD. The pressure generating and regulating system 220 and control circuitry (e.g., including a processor) 230 may generate, measure, and regulate pneumatic pressure that inflates or deflates the inflatable bladder 212, and may further comprise such elements as a pump, a valve, a pressure sensor, and/or other suitable elements, as previously described with reference to blood pressure measurement controller 120. In particular, pressure generating and regulating system 220 in cooperation with control circuitry 230 may be configured to implement a volume clamp method with the finger cuff 104 by: applying pneumatic pressure to the inflatable bladder 212 of the finger cuff 104 to replicate the patient's blood pressure based upon measuring the pleth signal received from the LED-PD pair 214 of the finger cuff 104 (e.g., to keep the pleth signal constant); and measuring the patient's blood pressure by monitoring the pressure of the inflatable bladder 212 based upon input from a pressure sensor, which should be related to or the same as patient's blood pressure, and may further command the display of the patient's blood pressure on the patient monitoring device. Furthermore, the control circuitry 230 may be configured to subtract out the gravity generated pressure differences between the patient's heart level and finger level as measured by the HRS 134 in calculating the patient's blood pressure.

Optional examples may relate to utilizing non-plasticized or chemically inert tubing 136 as the tubing 136 between the HRS 134 at the patient's heart level and a pressure sensor of the HRS coupled to the blood pressure measurement controller 120 of the finger cuff system 102, as will be described in more detail hereafter. A more resilient HRS system used with a finger cuff system may be achieved by utilizing non-plasticized tubing or chemically inert tubing. In particular, an HRS 134 used with a finger cuff system that incorporates non-plasticized tubing 136, chemically inert tubing 136, chemically resistant PVC tubing 136, or PVC tubing having a chemically resistant liner 136, has improved signal stability over long periods of time. By incorporating this type of tubing to contain oil (e.g., bioblend oil), bulging of the bladder of the HRS 134 at the patient's heart level may be prevented, which causes a rise in pressure over time that causes drift and difficulty in the zeroing functions of the HRS 134. As has been described, plasticizer from PVC tubing migrates into bioblend oil, causing excess oil-plasticizer mixture inside the tubing. The use of non-plasticized or chemically inert tubing maintains a constant amount of oil that fills the tubing thus providing a measurable stable signal over extended periods of time for the HRS.

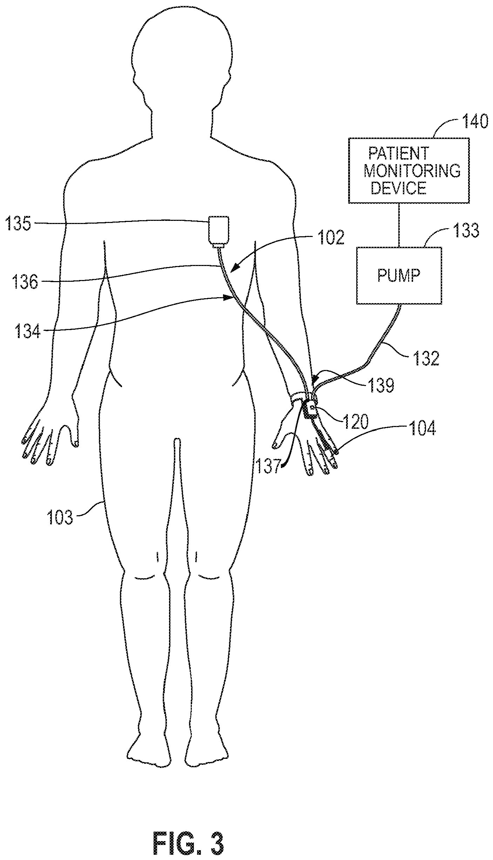

With additional reference to FIG. 3, as can be seen in FIG. 3, as one optional example, a finger cuff blood pressure measurement system to measure the blood pressure of a patient 103 and to prevent swelling of the tubing may comprise: a heart reference sensor (HRS) 134; a finger cuff 104 attachable to a patient's finger; and a blood pressure measurement controller 120 (e.g., attached to a patient's wrist), as has been previously described.

In particular, as an optional example, the HRS 134 may include first portion 135 that includes a housing that contains a bladder for mounting proximate the patient's heart level, tubing 136, and a pressure sensor 137 that is coupled to the blood pressure measurement controller 120. As an optional example, the finger cuff 104 may be attached to a patient's finger and the first portion 135 of the HRS 134 may be mounted proximate the patient's heart level, in which, the first portion of the HRS 134 includes a bladder. Further, a second portion 139 of the HRS includes the pressure sensor 137 that is mounted proximate to the finger cuff 104. As an optional example, the pressure sensor 137 may be mounted to the same finger as the finger cuff or a different finger, or may be directly mounted to the blood pressure measurement controller 120. Therefore, the HRS 134 includes a first portion 135 including a bladder mounted proximate the patient's heart level, tubing 136, and a second portion 139 including the pressure sensor 137.

In particular, as has been described, in one optional example, the blood pressure measurement controller 120 is coupled to the HRS 134 and the finger cuff 104 to perform blood pressure measurement functions. The pressure sensor 137 may be located at approximately the same height level of the finger cuff 104. Further, the tubing 136 extends from the top first portion 135 of the HRS 134 including the HRS bladder to the pressure sensor 137. The HRS bladder includes liquid in fluid communication with liquid in the tube 136 connected to pressure sensor 137 at the patient's finger level, such that, gravity generated pressure differences between the patient's heart level and finger level are measured by the pressure sensor 137 of the HRS 134, to be subtracted from the blood pressure measurement processed by the blood pressure measurement controller 120.

As has been described, as one optional example, the blood pressure measurement controller 120 utilizing the volume clamp method: controls the pneumatic pressure applied to the bladder of finger cuff 104 (e.g., from a pump 133) to replicate the patient's blood pressure based upon measuring the pleth signal received from the LED-PD pair of the finger cuff 104 (e.g., to keep the pleth signal constant); measures the patient's blood pressure by monitoring the pressure of the bladder of the finger cuff; and subtracts out the gravity generated pressure differences between the patient's heart level and finger level as measured by the pressure sensor 137 of the HRS 134 in calculating the patient's blood pressure. Further, in one optional example, the patient monitoring device 140 may display physiological readings/data of a patient including blood pressure, as well as any other suitable physiological patient readings.

As an optional example, it should be appreciated that in operation, a healthcare provider may: attach the finger cuff 104 to the patient's finger; attach the blood pressure measurement controller 120 to the patient's wrist; mount the top first portion 135 of the HRS 134 proximate the patient's heart level; and attach or mount the pressure sensor 137 of the second portion 139 of the HRS to the blood pressure measurement controller 120 or to the patient's finger. It should be appreciated that this is merely an optional example of setting up the system and many other operations and ordering of operations are possible.

According to the invention the tubing 136 is non-plasticized tubing or chemically inert tubing. As one optional example, the non-plasticized or chemically inert tubing 136 may include a flexible polymer. This type of chemically inert flexible polymer tubing may include at least one or more of: polyurethane, polyolefin, polyester, co-polyester, polyamide, co-polyamide, or fluoropolymer. As another optional example, the non-plasticized tube 136 may be flexible PVC tube lined with a flexible polymer. The flexible polymer lining of the PVC tube may include at least one or more of: polyurethane, polyolefin, polyester, co-polyester, polyamide, co-polyamide, or fluoropolymer.

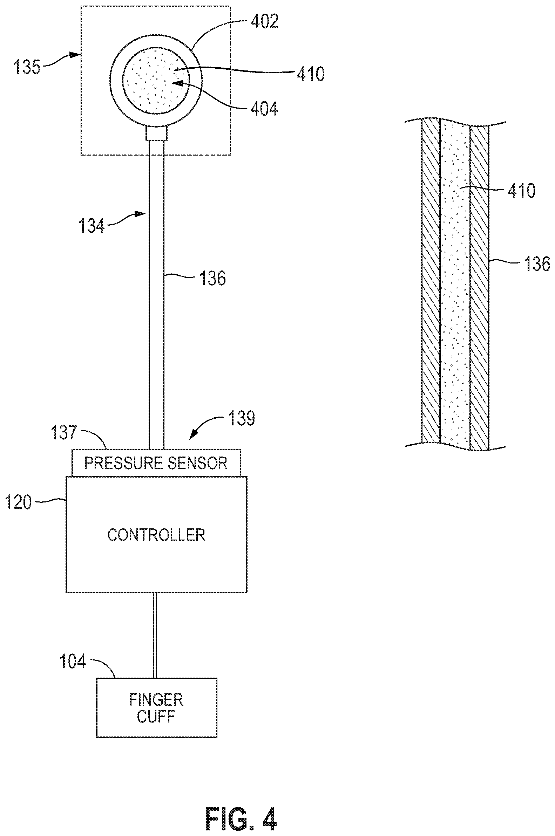

With additional reference to FIG. 4, an optional example of the HRS 134 utilizing non-plasticized tubing 136 or chemically inert tubing 136 will be described. As can be seen in this optional example, a bladder 402 of the first top portion 135 of HRS 134 is shown connected by tubing 136 including oil 410 to the pressure sensor 137 that is coupled to blood pressure measurement controller 120 that is further coupled to the finger cuff 104. The bladder 402 is approximately circular shape including a circular reservoir 404 that includes oil 410 in fluid communication with the oil 410 of the tubing 136. In this optional example, the tubing 136 may be non-plasticized tubing or chemically inert tubing. For example, the chemically inert tubing 136 may include a flexible polymer. This type of chemically inert flexible polymer tubing may include at least one or more of: polyurethane, polyolefin, polyester, co-polyester, polyamide, co-polyamide, or fluoropolymer.

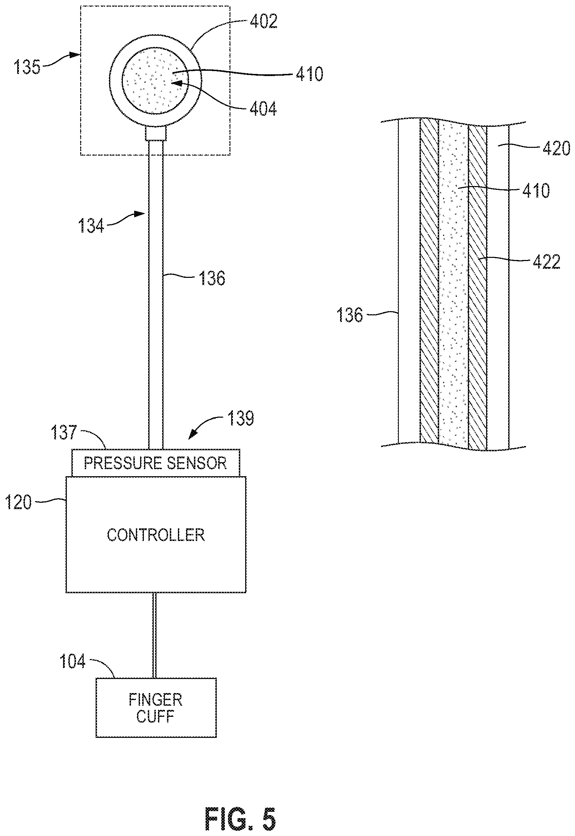

With additional reference to FIG. 5, an optional example of the HRS 134 utilizing non-plasticized tubing 136 including a flexible PVC tube lined with a flexible polymer will be described. As can be seen in this example, a bladder 402 of the first top portion 135 of HRS 134 is shown connected by tubing 136 including oil 410 to the pressure sensor 137 that is coupled to blood pressure measurement controller 120 that is further coupled to the finger cuff 104. The bladder 402 is approximately circular shape including a circular reservoir 404 that includes oil 410 in fluid communication with the oil 410 of the tubing 136. In this optional example, the tubing 136 may be a flexible PVC tube 420 lined with a flexible polymer 422. The flexible polymer lining 422 may include at least one or more of polyurethane, polyolefin, polyester, co-polyester, polyamide, co-polyamide, or fluoropolymer.

It should be appreciated that the optional examples provided in FIGs. 4-5 are merely optional examples of different types of tubing that can be used in a finger cuff system that utilizes an HRS. Also, as one optional example, the pressure sensor 137 may be mounted to the same finger as the finger cuff 104 of a patient or a different finger, or may be directly mounted to the blood pressure measurement controller 120 on the patient's wrist. Further, the optional examples of the different types of flexible polymers that may be utilized are merely examples and it should be appreciated that different types of flexible polymers may be utilized. Moreover, it should be appreciated that the previously described optional example of the chemically inert or non-plasticized tubing including a flexible polymer (with the previously described example chemicals) or the optional example of the non-plasticized tubing including a flexible PVC tube lined with a flexible polymer (with the previously described example chemicals), may each be used independently or may be used in combination with one another and may be used in combination with other optional examples.

In particular, as described above, the blood pressure measurement controller 120 is coupled to the HRS 134 and the finger cuff 104 to perform blood pressure measurement functions. The pressure sensor 137 may be located at approximately the same height level of the finger cuff 104. Further, the tubing 136 extends from the top first portion 135 of the HRS 134 including the HRS bladder 402 to the pressure sensor 137. The HRS bladder 402 includes oil in fluid communication with oil in the tube 136 connected to pressure sensor 137 at the patient's finger level, such that, gravity generated pressure differences between the patient's heart level and finger level are measured by the pressure sensor 137 of the HRS 134, to be subtracted from the blood pressure measurement processed by the blood pressure measurement controller 120.

By incorporating non-plasticized or chemically inert tubing 136 to contain oil between the bladder 402 of the HRS 134 at the patient's heart level and the pressure sensor 137 of the HRS 134, a more resilient HRS system used with a finger cuff system may be achieved. In particular, as set forth in the optional examples, an HRS 134 used with a finger cuff system that incorporates non-plasticized tubing 136, chemically inert tubing 136, chemically resistant PVC tubing 136, or PVC tubing having a chemically resistant liner 136, has improved signal stability over long periods of time. By incorporating this type of tubing to contain oil (e.g., bioblend oil), bulging of the bladder of the HRS 134 at the patient's heart level may be prevented, which causes a rise in pressure over time that causes drift and difficulty in the zeroing functions of the HRS 134. As has been described, plasticizer from PVC tubing migrates into bioblend oil, causing excess oil-plasticizer mixture inside the tubing. The use of non-plasticized or chemically inert tubing maintains a constant amount of oil that fills the tubing thus providing a measurable stable signal over extended periods of time for the HRS.

It should be appreciated that aspects of the invention previously described may be implemented in conjunction with the execution of instructions or code by processors, circuitry, controllers, control circuitry, etc. (e.g., processor, circuitry, etc., of the blood pressure measurement controller). As an example, control circuitry may operate under the control of a program, algorithm, code, routine, or the execution of instructions to execute methods or processes in accordance with embodiments of the invention previously described. For example, such a program may be implemented in firmware or software (e.g. stored in memory and/or other locations) and may be implemented by processors, control circuitry, and/or other circuitry, these terms being utilized interchangeably. Further, it should be appreciated that the terms processor, microprocessor, circuitry, control circuitry, circuit board, controller, microcontroller, etc., refer to any type of logic or circuitry capable of executing logic, commands, instructions, software, firmware, functionality, etc., which may be utilized to execute embodiments of the invention.

The various illustrative logical blocks, processors, modules, and circuitry described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, non-transitory computer readable medium, or any other form of storage medium known in the art. An exemplary storage medium is coupled to the processor such the processor can read information from, and write information to, the storage medium. In the alternative, the storage medium may be integral to the processor.

The previous description of the disclosed optional examples is provided to enable any person skilled in the art to make or use the present invention. Various modifications to these optional examples will be readily apparent to those skilled in the art. Thus, the present invention is not intended to be limited to the optional examples shown, but limited only by the appended claims.

## Claims

1. A finger cuff blood pressure measurement system to measure the blood pressure of a patient (103) comprising:
a heart reference sensor (134), hereinafter HRS, including a bladder for mounting proximate the patient's heart level, tubing (136), and a pressure sensor (137);
a finger cuff (104) attachable to a patient's finger; and
a blood pressure measurement controller (120) coupled to the HRS (134) and the finger cuff (104) to perform blood pressure measurement functions; wherein
the pressure sensor (104) is to be located at approximately the same height level of the finger cuff (104), and the tubing (136) extends from the bladder of the HRS to the pressure sensor (137);
the bladder of the HRS (134) includes liquid in fluid communication with liquid in the tubing connected to the pressure sensor (137) at the patient's finger level such that gravity-generated pressure differences between the patient's heart level and the finger level are measurable by the pressure sensor (137) of the HRS and to be subtracted from the blood pressure measurement processed by the blood pressure measurement controller (120); **characterized in that**
the tubing (136) is non-plasticized tubing or chemically inert tubing.

2. The finger cuff blood pressure measurement system of claim 1, wherein the chemically inert or non-plasticized tubing (136) includes a flexible polymer.

3. The finger cuff blood pressure measurement system of claim 2, wherein the flexible polymer includes one of polyurethane, polyolefin, polyester, copolyester, polyamide, co-polyamide, or fluoropolymer.

4. The finger cuff blood pressure measurement system of any of the claims 1 to 3, wherein the non-plasticized tubing (136) is a flexible PVC tube with a flexible polymer lining (422) on the inner diameter.

5. The finger cuff blood pressure measurement system of claim 4, wherein the flexible polymer lining (422) of the PVC tube includes one of polyurethane, polyolefin, polyester, co-polyester, polyamide, co-polyamide, or fluoropolymer.

6. The finger cuff blood pressure measurement system of any of the claims 1 to 5, wherein the liquid includes a bioblend oil.

7. The finger cuff blood pressure measurement system of any of the claims 1 to 6, wherein the finger cuff includes an enclosing portion that encloses the patient's finger, the enclosing portion including a finger cuff bladder and a light emitting diode (LED) and photodiode (PD) pair; and wherein
the blood pressure measurement controller is coupled to the HRS and the finger cuff to perform blood pressure measurement functions based upon interaction with the bladder and the LED-PD pair of the finger cuff utilizing a volume clamp method.

8. A method for measuring the blood pressure of a patient utilizing a finger cuff blood pressure measurement system including a finger cuff and a blood pressure measurement controller, the method comprising:
attaching a finger cuff (104) to the patient's finger;
mounting a first portion of a heart reference sensor (134), hereinafter HRS, to the patient proximate the patient's heart level, the first portion of the HRS (134) including a bladder; and
mounting a second portion of the HRS proximate the finger cuff (104), the second portion of the HRS including a pressure sensor (137); wherein
tubing (136) is connected between the bladder of the HRSand the pressure sensor (137) of the HRS (134);
a blood pressure measurement controller (120) is coupled to the HRS (134) and the finger cuff (104) to perform blood pressure measurement functions; and wherein
the pressure sensor (137) is mounted at approximately the same height level of the finger cuff (104);
the tubing extends from the bladder to the pressure sensor (137) of the HRS (134),
the bladder of the HRS includes liquid in fluid communication with liquid in the tubing (134) connected to the pressure sensor (137) at the patient's finger level such that gravity generated pressure differences between the patient's heart level and finger level are measurable by the pressure sensor (137) of the HRS to be subtracted from the blood pressure measurement processed by the blood pressure measurement controller (120), and wherein
the tubing is non-plasticized tubing or chemically inert tubing.

9. The method of claim 8, wherein the chemically inert or non-plasticized tubing includes a flexible polymer.

10. The method of claim 9, wherein the flexible polymer includes one of polyurethane, polyolefin, polyester, co-polyester, polyamide, co-polyamide, or fluoropolymer.

11. The method of any of the claims 8 to 10, wherein the non-plasticized tube is a flexible PVC tube lined with a flexible polymer.

12. The method of claim 11, wherein the flexible polymer lining of the PVC tube includes one of polyurethane, polyolefin, polyester, co-polyester, polyamide, co-polyamide, or fluoropolymer.

13. The method of any of the claims 8 to 12, wherein the liquid includes a bioblend oil.

## Patentansprüche

1. Fingermanschetten-Blutdruckmesssystem zum Messen des Blutdrucks eines Patienten (103), umfassend:
einen Herzreferenzsensor (134), nachfolgend HRS, der eine Blase zur Anbringung nahe dem Herzniveau des Patienten, Schlauchmaterial (136) und einen Drucksensor (137) einschließt;
eine Fingermanschette (104), die an einem Finger eines Patienten befestigbar ist; und
eine Blutdruckmesssteuerung (120), die an den HRS (134) und die Fingermanschette (104) gekoppelt ist, um Blutdruckmessfunktionen durchzuführen; wobei
der Drucksensor (104) annähernd auf dem gleichen Höhenniveau der Fingermanschette (104) zu positionieren ist, und das Schlauchmaterial (136) sich von der Blase des HRS zu dem Drucksensor (137) erstreckt;
die Blase des HRS (134) Flüssigkeit in Fluidkommunikation mit Flüssigkeit in dem Schlauchmaterial einschließt, das mit dem Drucksensor (137) auf Höhe des Patientenfingers verbunden ist, so dass schwerkrafterzeugte Druckdifferenzen zwischen dem Herzniveau des Patienten und dem Fingerniveau durch den Drucksensor (137) des HRS messbar sind und von der Blutdruckmessung zu subtrahieren sind, die von der Blutdruckmesssteuerung (120) verarbeitet wird; **dadurch gekennzeichnet, dass**
das Schlauchmaterial (136) nicht-plastifiziertes Schlauchmaterial oder chemisch inertes Schlauchmaterial ist.

2. Fingermanschetten-Blutdruckmesssystem nach Anspruch 1, wobei das chemisch inerte oder nicht-plastifizierte Schlauchmaterial (136) ein flexibles Polymer einschließt.

3. Fingermanschetten-Blutdruckmesssystem nach Anspruch 2, wobei das flexible Polymer eines von Polyurethan, Polyolefin, Polyester, Copolyester, Polyamid, Copolyamid oder Fluorpolymer einschließt.

4. Fingermanschetten-Blutdruckmesssystem nach einem der Ansprüche 1 bis 3, wobei das nicht-plastifizierte Schlauchmaterial (136) ein flexibler PVC-Schlauch mit einer flexiblen Polymerauskleidung (422) auf dem Innendurchmesser ist.

5. Fingermanschetten-Blutdruckmesssystem nach Anspruch 4, wobei die flexible Polymerauskleidung (422) des PVC-Schlauchs eines von Polyurethan, Polyolefin, Polyester, Copolyester, Polyamid, Copolyamid oder Fluorpolymer einschließt.

6. Fingermanschetten-Blutdruckmesssystem nach einem der Ansprüche 1 bis 5, wobei die Flüssigkeit ein Bioblend-Öl einschließt.

7. Fingermanschetten-Blutdruckmesssystem nach einem der Ansprüche 1 bis 6, wobei die Fingermanschette einen umschließenden Abschnitt einschließt, der den Finger des Patienten umschließt, wobei der umschließende Abschnitt eine Fingermanschettenblase und ein Paar aus lichtemittierender Diode (LED) und Photodiode (PD) einschließt; und wobei
die Blutdruckmesssteuerung an den HRS und die Fingermanschette gekoppelt ist, um Blutdruckmessfunktionen basierend auf Interaktion mit der Blase und dem Paar aus LED und PD der Fingermanschette durchzuführen, wobei eine Volume-Clamp-Methode genutzt wird.

8. Verfahren zum Messen des Blutdrucks eines Patienten, wobei ein Fingermanschetten-Blutdruckmesssystem genutzt wird, das eine Fingermanschette und eine Blutdruckmesssteuerung einschließt, wobei das Verfahren umfasst:
Befestigen einer Fingermanschette (104) am Finger des Patienten;
Anbringen eines ersten Anteils eines Herzreferenzsensors (134), nachfolgend HRS, an dem Patienten nahe dem Herzniveau des Patienten, wobei der erste Anteil des HRS (134) eine Blase einschließt; und
Anbringen eines zweiten Anteils des HRS nahe der Fingermanschette (104), wobei der zweite Anteil des HRS einen Drucksensor (137) einschließt; wobei
Schlauchmaterial (136) zwischen der Blase des HRS und dem Drucksensor (137) des HRS (134) verbunden ist;
eine Blutdruckmesssteuerung (120) an den HRS (134) und die Fingermanschette (104) gekoppelt ist, um Blutdruckmessfunktionen durchzuführen; und wobei
der Drucksensor (137) auf annähernd dem gleichen Höhenniveau der Fingermanschette (104) montiert wird;
das Schlauchmaterial sich von der Blase zu dem Drucksensor (137) des HRS (134) erstreckt,
die Blase des HRS Flüssigkeit in Fluidkommunikation mit Flüssigkeit in dem Schlauchmaterial (134), das mit dem Drucksensor (137) verbunden ist, auf Höhe des Patientenfingers einschließt, so dass schwerkrafterzeugte Druckdifferenzen zwischen dem Herzniveau des Patienten und dem Fingerniveau durch den Drucksensor (137) des HRS messbar sind und von der Blutdruckmessung zu subtrahieren sind, die von der Blutdruckmesssteuerung (120) verarbeitet wird; und wobei
das Schlauchmaterial nicht-plastifiziertes Schlauchmaterial oder chemisch inertes Schlauchmaterial ist.

9. Verfahren nach Anspruch 8, wobei das chemisch inerte oder nicht-plastifizierte Schlauchmaterial ein flexibles Polymer einschließt.

10. Verfahren nach Anspruch 9, wobei das flexible Polymer eines von Polyurethan, Polyolefin, Polyester, Copolyester, Polyamid, Copolyamid oder Fluorpolymer einschließt.

11. Verfahren nach einem der Ansprüche 8 bis 10, wobei der nicht-plastifizierte Schlauch ein flexibler PVC-Schlauch ist, der mit einem flexiblen Polymer ausgekleidet ist.

12. Verfahren nach Anspruch 11, wobei die flexible Polymerauskleidung des PVC-Schlauchs eines von Polyurethan, Polyolefin, Polyester, Copolyester, Polyamid, Copolyamid oder Fluorpolymer einschließt.

13. Verfahren nach einem der Ansprüche 8 bis 12, wobei die Flüssigkeit ein Bioblend-Öl einschließt.

## Revendications

1. Système de mesure de tension artérielle à doigtier destiné à mesurer la tension artérielle d'un patient (103) comprenant :
un capteur cardiaque de référence (134), ci-après HRS, comprenant une poche destinée à être montée à proximité du niveau du coeur du patient, une tubulure (136) et un capteur de pression (137) ;
un doigtier (104) pouvant être fixé au doigt d'un patient ; et
un dispositif de commande de mesure de tension artérielle (120) accouplé au HRS (134) et au doigtier (104) destiné à effectuer des fonctions de mesure de tension artérielle ;
le capteur de pression (104) étant destiné à être situé approximativement au même niveau de hauteur que celui du doigtier (104) et la tubulure (136) s'étendant depuis la poche du HRS au capteur de pression (137) ;
la poche du HRS (134) comprenant un liquide en communication fluidique avec le liquide dans la tubulure reliée au capteur de pression (137) au niveau du doigt du patient de telle sorte que des différences de pression générées par gravité entre le niveau du coeur du patient et le niveau de doigt sont mesurables par le capteur de pression (137) du HRS et à soustraire de la mesure de tension artérielle traitée par le dispositif de commande de mesure de tension artérielle (120) ; **caractérisé en ce que**
la tubulure (136) est une tubulure non plastifiée ou une tubulure chimiquement inerte.

2. Système de mesure de tension artérielle à doigtier selon la revendication 1, la tubulure chimiquement inerte ou non plastifiée (136) comprenant un polymère souple.

3. Système de mesure de tension artérielle à doigtier selon la revendication 2, le polymère souple comprenant un polymère parmi le polyuréthane, une polyoléfine, le polyester, un copolyester, le polyamide, un copolyamide ou un fluoropolymère.

4. Système de mesure de tension artérielle à doigtier selon l'une quelconque des revendications 1 à 3, la tubulure non plastifiée (136) étant une tubulure en PVC souple pourvue d'un revêtement polymère souple (422) sur le diamètre interne.

5. Système de mesure de tension artérielle à doigtier selon la revendication 4, le revêtement polymère souple (422) de la tubulure en PVC comprenant un polymère parmi le polyuréthane, une polyoléfine, le polyester, un copolyester, le polyamide, un copolyamide ou un fluoropolymère.

6. Système de mesure de tension artérielle à doigtier selon l'une quelconque des revendications 1 à 5, le liquide comprenant une bio-huile mixte.

7. Système de mesure de tension artérielle à doigtier selon l'une quelconque des revendications 1 à 6, le doigtier comprenant une partie d'enveloppement qui renferme le doigt du patient, la partie d'enveloppement comprenant une poche de doigtier et une paire de diode électroluminescente (DEL) et de photodiode (PD) ; et le dispositif de commande de mesure de tension artérielle étant accouplé au HRS et au doigtier pour effectuer des fonctions de mesure de tension artérielle sur la base d'une interaction avec la poche et la paire de DEL-PD du doigtier à l'aide d'un procédé de serrage volumique.

8. Procédé de mesure de tension artérielle d'un patient à l'aide d'un système de mesure de tension artérielle à doigtier comprenant un doigtier et un dispositif de commande de mesure de tension artérielle, le procédé comprenant :
la fixation d'un doigtier (104) au doigt du patient ;
le montage d'une première partie d'un capteur cardiaque de référence (134), ci-après HRS, au patient à proximité du niveau du coeur du patient, la première partie du HRS (134) comprenant une poche ; et
le montage d'une seconde partie du HRS à proximité du doigtier (104), la seconde partie du HRS comprenant un capteur de pression (137) ;
la tubulure (136) étant reliée entre la poche du HRS et le capteur de pression (137) du HRS (134) ;
un dispositif de commande de mesure de tension artérielle (120) étant accouplé au HRS (134) et au doigtier (104) pour effectuer des fonctions de mesure de tension artérielle ; et
le capteur de pression (137) étant monté approximativement au même niveau de hauteur que celui du doigtier (104) ;
la tubulure s'étendant depuis la poche au capteur de pression (137) du HRS (134),
la poche du HRS comprenant un liquide en communication fluidique avec le liquide dans la tubulure (134) reliée au capteur de pression (137) au niveau du doigt du patient de telle sorte que des différences de pression générées par gravité entre le niveau du coeur et le niveau du doigt du patient sont mesurables par le capteur de pression (137) du HRS et à soustraire de la mesure de tension artérielle traitée par le dispositif de commande de mesure de tension artérielle (120) et
la tubulure étant une tubulure non plastifiée ou une tubulure chimiquement inerte.

9. Procédé selon la revendication 8, la tubulure chimiquement inerte ou non plastifiée comprenant un polymère souple.

10. Procédé selon la revendication 9, le polymère souple comprenant un polymère parmi le polyuréthane, une polyoléfine, le polyester, un copolyester, le polyamide, un copolyamide ou un fluoropolymère.

11. Procédé selon l'une quelconque des revendications 8 à 10, la tubulure non plastifiée étant une tubulure en PVC souple revêtue d'un polymère souple.

12. Procédé selon la revendication 11, le revêtement polymère souple de la tubulure en PVC comprenant un polymère parmi le polyuréthane, une polyoléfine, le polyester, un copolyester, le polyamide, un copolyamide ou un fluoropolymère.

13. Procédé selon l'une quelconque des revendications 8 à 12, le liquide comprenant une bio-huile mixte.
